# EUROPEAN PATENT APPLICATION

(11) **EP 4 119 146 A1**
(43) Date of publication of application: **18.01.2023**
(21) Application number: 21768167.5
(22) Date of filing: 09.02.2021
(51) Int. Cl.: A61K 31/7048, A61K 31/34, A61P 31/12

(54) **3CLPRO-TARGETING PHILLYRIN, DERIVATIVE THEREOF, AND USE THEREOF AGAINST NOVEL CORONAVIRUS**

(30) Priority: 09.03.2020 CN 202010155743
(71) Applicant: Dalian Fusheng Natural Medicine Development Co., Ltd., Dalian, Liaoning 116100 (CN)
(72) Inventor: YANG, Zifeng, Dalian, Liaoning 116100 (CN); FU, Li, Dalian, Liaoning 116100 (CN); JIANG, Ge, Dalian, Liaoning 116100 (CN); ZHONG, Nanshan, Dalian, Liaoning 116100 (CN); YANG, Wei, Dalian, Liaoning 116100 (CN); MA, Qinhai, Dalian, Liaoning 116100 (CN); HUI, Min, Dalian, Liaoning 116100 (CN); LI, Chufang, Dalian, Liaoning 116100 (CN); WANG, Shuo, Dalian, Liaoning 116100 (CN); PAN, Weiyi, Dalian, Liaoning 116100 (CN); LU, Qi, Dalian, Liaoning 116100 (CN); SONG, Aiai, Dalian, Liaoning 116100 (CN); HOU, Jirui, Dalian, Liaoning 116100 (CN); LI, Runfeng, Dalian, Liaoning 116100 (CN); LU, Mingming, Dalian, Liaoning 116100 (CN); WANG, Yingping, Dalian, Liaoning 116100 (CN); LIU, Yang, Dalian, Liaoning 116100 (CN); LIU, Guoyou, Dalian, Liaoning 116100 (CN); FU, Wenfei, Dalian, Liaoning 116100 (CN); FENG, Xue, Dalian, Liaoning 116100 (CN); ZHOU, Qingfeng, Dalian, Liaoning 116100 (CN); YI, Xiaofeng, Dalian, Liaoning 116100 (CN); LIN, Rongxin, Dalian, Liaoning 116100 (CN); ZHANG, Yu, Dalian, Liaoning 116100 (CN)
(74) Representative: Neilson, Martin Mark
(86) International application number: PCT/CN2021/076179
(87) International publication number: WO 2021/179878

(57) **Abstract**

The present invention provides a use of a phillyrin/phillygenin composition which targets and inhibits 3CLpro protein of COVID-19 virus, in preparation of an anti-coronavirus drug or a drug for treating a disease caused by the coronavirus, wherein the coronavirus is COVID-19 virus, and the disease caused by the coronavirus is COVID-19.

## Description

### TECHNICAL FIELD OF THE INVENTION

The present invention belongs to the technical field of medicine, more particularly, the present invention relates to use of phillyrin and/or its derivative(s) and a phillyrin/phillygenin composition which target and inhibit 3CLpro protein of COVID-19 virus, in preparation of a drug against coronavirus (particularly COVID-19 virus), and the like.

### BACKGROUND OF THE INVENTION

COVID-19 refers to the coronavirus disease 2019 (also known as the novel coronavirus pneumonia). The pathogen of COVID-19 is COVID-19 virus (also known as the novel coronavirus), which belongs to the coronavirus and has high homology with the SARS spread in 2003. There is currently no specific medicine for COVID-19. In fact, there is still no specific medicine for the SARS spread previously.

The present inventors have focused on studying Chinese herb extracts and have accidentally and surprisingly discovered that a pharmaceutical composition including phillyrin and a small amount of phillygenin has a synergistic medicinal effect, even an interactively synergistic medicinal effect, in terms of antivirus effect. For example, Chinese patent application CN105362283A discloses a phillyrin/phillygenin composition and its use in alleviating or/and treating viral diseases, wherein the viral diseases are those caused by influenza virus, parainfluenza virus, coxsackievirus CoxA16, respiratory syncytial virus RSV, herpes simplex virus HSV-I, herpes simplex virus HSV-II, herpes simplex virus CVB3, adenovirus ADV or enterovirus EV71.

However, there are many antiviral agents with diverse mechanisms of action, and it is not possible to predict which of the existing antiviral agents will inhibit the emerging COVID-19 virus. In particular, since coronavirus is quite different from the viruses targeted by the above-mentioned phillyrin/phillygenin composition, and the study on COVID-19 virus and its therapeutic mechanism are still inconclusive at present, those skilled in the art cannot predict whether a phillyrin/phillygenin composition has an inhibitory effect on coronavirus (particularly COVID-19 virus).

In addition, the effects of phillyrin, phillygenin or their derivatives on hydrolase of COVID-19 virus (3-chymotrypsin-like cysteine protease, abbreviated as 3CL pro protein) have not been reported.

However, the present inventors accidentally found that the phillyrin/phillygenin composition has an excellent inhibitory effect on COVID-19 virus, and in consideration of its high safety, its clinical approval for other indications is nearly completed. Therefore, it has a prospect of being a drug for treating COVID-19 and may quickly come into use in clinical practices. Further, the present inventors found that phillyrin, phillygenin or their derivatives have a targeting inhibitory effect on the 3CL pro protein of COVID-19 virus and can act as an inhibitor of the protein.

### SUMMARY OF THE INVENTION

The technical problem to be solved by the present invention is to provide a novel agent for targeting inhibition of 3CLpro protein of COVID-19 virus, and to provide a drug against coronavirus (particularly COVID-19 virus) or a drug for treating a disease caused by the coronavirus (particularly COVID-19).

Particularly, in a first aspect, the present invention provides a use of phillyrin and/or its derivative(s) in preparation of an agent for inhibiting 3CLpro protein of COVID-19 virus. The agent for inhibiting 3CLpro protein of C0VID-19 virus includes phillyrin and/or its derivative(s) thereof.

The use according to the first aspect of the present invention may be single use of phillyrin or its derivative(s), or combined use of phillyrin and its derivative(s), for example, use of a phillyrin/ phillygenin composition. In the use according to the first aspect of the present invention, the inhibition may be in vivo or in vitro.

In the use according to the first aspect of the present invention, the derivative is preferably KD-2-GLU or KD-2-SO₃H.

Accordingly, in a second aspect, the present invention also provides a method for inhibiting 3CLpro protein of COVID-19 virus, including a step of contacting 3CLpro protein of COVID-19 virus with phillyrin and/or its derivative(s). The method may be an in vivo or in vitro.

In the method according to the second aspect of the present invention, the derivative is preferably KD-2-GLU or KD-2-SO₃H.

In a third aspect, the present invention provides a use of a phillyrin/phillygenin composition in preparation of a drug against coronavirus. In addition, the present invention also provides a use of a phillyrin/phillygenin composition in preparation of a drug for treating disease(s) caused by coronavirus. The action against coronavirus may be an action against coronavirus in vitro, e.g., inhibition of coronavirus proliferation in vitro, but the action against coronavirus is preferably in vivo, that is, treatment of coronavirus-induced diseases.

Herein, terms "phillyrin/phillygenin", "phillyrin and phillygenin" and "phillyrin and phillygenin composition" may be used interchangeably, and mean a composition consisting of phillyrin and phillygenin, i.e. phillyrin/phillygenin is considered as a whole, unless otherwise indicated. In the present invention, preferably, the weight ratio of phillyrin to phillygenin is 2~98: 2~98, preferably 80~98: 2~20, and more preferably 90~98: 2∼10, for example, 90: 10 or 98: 2.

In the use according to the third aspect of the present invention, the coronavirus is preferably COVID-19 virus, or the disease caused by the coronavirus is preferably COVID-19. Preferably, the phillyrin/phillygenin composition inhibits 3CLpro protein of COVID-19 virus, that is, in the use according to the third aspect of the present invention, the phillyrin/phillygenin composition acts against COVID-19 virus or treats disease(s) caused by COVID-19 virus by inhibiting 3CLpro protein of COVID-19 virus.

The phillyrin/phillygenin composition may be used in combination with other drug(s) against coronavirus (e.g., COVID-19 virus) or treating disease(s) caused by coronavirus (e.g., COVID-19), or may be used alone. The present inventors found that the mechanism of the phillyrin/phillygenin composition against COVID-19 virus includes inhibition of 3CLpro protein of COVID-19 virus.

The use according to the third aspect of the present invention is preferably use of the phillyrin/phillygenin composition as a sole (single) pharmaceutical active ingredient, that is, the sole active pharmaceutical ingredient in the drug is the phillyrin/phillygenin composition. That is, the third aspect of the present invention preferably provides a use of the phillyrin/phillygenin composition as the sole active pharmaceutical ingredient in preparation of a drug against coronavirus (e.g., COVID-19 virus), or use of the phillyrin/phillygenin composition as the sole active pharmaceutical ingredient in preparation of a drug for treating disease(s) caused by coronavirus (e.g., COVID-19).

The drug may include a pharmaceutically acceptable carrier so that a pharmaceutical formulation may be prepared. This is well known to those skilled in the art. In the use according to the third aspect of the present invention, said drug is preferably present in a form of tablet, capsule, pill, powder, granule, syrup, solution, emulsion, injection, spray, aerosol, gel, cream, cataplasmata, adhesive plaster or emplastrum.

Accordingly, the present invention also provides second medicinal use of a phillyrin/phillygenin composition and a treating method using the same. In a fourth aspect, the present invention provides a phillyrin/phillygenin composition against coronavirus (e.g., COVID-19 virus). The present invention also provides a phillyrin/phillygenin composition for treating disease(s) caused by coronavirus (e.g., COVID-19); alternatively, the present invention also provides a phillyrin/phillygenin composition for inhibiting 3CLpro protein of COVID-19 virus.

Further, the present invention provides a drug including a phillyrin/phillygenin composition against coronavirus (e.g., COVID-19 virus). The present invention also provides a drug including a phillyrin/phillygenin composition for treating disease(s) caused by coronavirus (e.g., COVID-19); alternatively, the invention also provides a drug including a phillyrin/phillygenin composition for inhibiting 3CLpro protein of COVID-19 virus.

Preferably, phillyrin/phillygenin is a sole active pharmaceutical ingredient in the drug including a phillyrin/phillygenin composition.

Also, preferably, in the drug including a phillyrin/phillygenin composition, said drug is present in a form of tablet, capsule, pill, powder, granule, syrup, solution, emulsion, injection, spray, aerosol, gel, cream, cataplasmata, adhesive plaster or emplastrum.

In a fifth aspect, the present invention provides a method against coronavirus (e.g., COVID-19 virus), including administrating an effective amount of a phillyrin/phillygenin composition to a patient in need. Accordingly, the present invention also provides a method for treating disease(s) caused by coronavirus (e.g., COVID-19), including administrating an effective amount of a phillyrin/phillygenin composition to a patient in need. Preferably, the phillyrin/phillygenin composition acts against COVID-19 virus or treats disease(s) caused by COVID-19 virus by inhibiting 3CLpro protein of COVID-19 virus.

Further, the present invention provides a method against COVID-19 virus, including administrating an effective amount of a drug including a phillyrin/phillygenin composition to a patient in need. Accordingly, the present invention also provides a method for treating disease(s) caused by coronavirus (e.g., COVID-19), including administrating an effective amount of a drug including a phillyrin/phillygenin composition to a patient in need.

Herein, the dosage (effective amount) and route of administration are generally determined by a doctor according to the specific condition of a patient (e.g., age, weight, gender, duration of illness, physical condition, severity of infection, etc.). As the specific condition of the patient may vary, the dosage of administration will also vary, and an appropriate amount may be selected within the scope of clinician's competence. The route of administration is determined according to the dosage form of the pharmaceutical composition, and suitable routes of administration include oral, parenteral, mucosal, intramuscular, intravenous, subcutaneous, intraocular, intradermal, transdermal, or the like. The administration route is preferably oral.

In the methods, preferably, the phillyrin/phillygenin composition is a sole active pharmaceutical ingredient in said drug.

In the methods, also preferably, said drug is present in a form of tablet, capsule, pill, powder, granule, syrup, solution, emulsion, injection, spray, aerosol, gel, cream, cataplasmata, adhesive plaster or emplastrum.

The present invention has following advantageous effects. An agent and a method for inhibiting 3CLpro protein of COVID-19 virus are provided. The phillyrin/phillygenin composition, even having a low concentration, can effectively inhibit coronavirus such as COVID-19 virus or the like, and the combination of phillyrin and phillygenin has a synergistic effect, which has a prospect of being a drug for treating coronavirus such as COVID-19 virus or the like and may quickly come into use in clinical practices.

For ease of understanding, the present invention will be hereinafter described in detail by referring to embodiments and drawings. It should be noted that these descriptions are only exemplary and do not constitute a limitation to the scope of the present invention. In accordance with the description, many of variations and changes of the present invention are apparent to those skilled in the art. Furthermore, published literature is cited by the present invention to more clearly describe the present invention. The full text of these published literature is incorporated herein by reference as if their full text has been repeated herein.

### BRIEF DESCRIPTION OF DRAWINGS

Figure 1 shows structures of phillyrin and its derivatives.
Figure 2 is a simulation diagram showing KD-1 binding to 3CLpro target protein of COVID-19.
Figure 3 is a simulation diagram showing KD-2-GLU binding to the COVID-19 target protein.
Figure 4 is a simulation diagram showing KD-2-SO₃H binding to the 3CLpro target protein of COVID-19.
Figure 5 shows results of cloning, expression and purification of 3CL pro protein.
Figure 6 is a UV analysis graph of 3CLpro protein and KD-1.
Figure 7 is a UV analysis graph of 3CLpro protein and KD2-GLU.
Figure 8 is a UV analysis graph of 3CLpro protein and KD2-SO₃H.

### DETAILED DESCRIPTION OF THE INVENTION

The invention is further exemplarily illustrated hereinafter by referring to embodiments. Unless specified, methods used in embodiments are all disclosed in technical literature of the field to which the present invention belongs and in the specification documents of drug regulatory agencies, and instruments, raw materials and reagents used are purchased from the open market.

### First Embodiment: Study on Phillyrin/Phillygenin Composition Acting against COVID-19 Virus in Vitro

**1 Test drug:** phillyrin/phillygenin composition, wherein the weight ratio of phillyrin to phillygenin is 90: 10 (Dalian Fusheng Natural Medicine Development Co., Ltd.).
**2 Cells:** VeroE6 cells (National Key Laboratory of Respiratory Diseases, Guangzhou Institute of Respiratory Health).
**3 Virus:** SARS-CoV-2 strain of COVID-19 virus (BSL-3 Laboratory of Guangzhou Customs Technology Center (High Pathogenicity Microorganism Laboratory, National Key Laboratory of Respiratory Diseases)), with a titer of TCID50 = 10⁻⁶/100 µL.
**4 Experimental procedures (all the experimental operations were performed in the BSL-3 laboratory):**
   (1) 100 µL of VeroE6 cells having a concentration of 2 × 10⁵ cells/mL was added to each well of a sterile 96-well culture plate, and incubated with 5% CO₂ at 37°C for 24 hours;
   (2) 100 TCID50 virus solution was added at 100 µL/well in the culture plate for the experimental group and the virus control group, and the virus was allowed to attach in a 37°C 5% CO₂ incubator for 2 hours;
   (3) After 2 hours, the cell culture medium in the 96-well culture plate was discarded; and the phillyrin/phillygenin composition was diluted into solutions having a series of concentrations as shown in Table 1. The solution was added into the well in 100 µl/well, in triplicate for each of the concentrations;
   (4) Cell control, blank control (solvent control) and virus control (negative control) were set up at the same time;
   (5) Cells were incubated in a 37°C 5% CO₂ incubator for 3-4 days;
   (6) Cytopathic effect (CPE) was observed using an optical microscope. The degree of CPE was recorded as the following 6 grades: "-" no CPE was observed; "±" cytopathic effect is less than 10%; "+" cytopathic effect is about 25%; "+ +" cytopathic effect is about 50%; "+ + +" cytopathic effect is about 75%; "+ + + +" cytopathic effect is more than 75%. The half effective concentration (IC50) was calculated using the Reed-Muench method or GraphPad Prism 5.0.
**5 Experimental results:**

As shown in Table 1-1, the phillyrin/phillygenin composition, even having a low concentration, can effectively inhibit COVID-19 virus. By calculation, the half maximal inhibitory concentration (IC₅₀) is 63.90 µg/mL (while the half maximal inhibitory concentration (IC₅₀) of pure phillyrin showed by the study at the same time is 179.1 µg/mL, and the inhibitory effect of pure phillygenin is worse). The combination of phillyrin and phillygenin in the composition has a synergistic effect, so it is expected to be used to treat COVID-19.

**Table 1-1**

| Drug concentration (µg/mL) | Inhibition (%) |
|---|---|
| 250.00 | 78.33±10.41 |
| 125.00 | 68.33±7.64 |
| 62.50 | 55.00±13.23 |
| 31.25 | 30.00±10.00 |
| 15.63 | 11.67±2.89 |

### Second Embodiment: Study on Phillyrin/Phillygenin Composition Acting Against HCoV-229E in Vitro

The coronavirus HCoV-229E is less pathogenic and usually causes only respiratory symptoms similar to the common cold. The present embodiment was performed generally by referring to the process shown in the first embodiment, except that the coronavirus used was HCoV-229E (BSL-3 Laboratory of Guangzhou Customs Technology Center (High Pathogenicity Microorganism Laboratory, National Key Laboratory of Respiratory Diseases)), with a titer of TCID50 = 10^{-5.5}/100 µL, and the titer of the virus used was 100TCID50; and the phillyrin/phillygenin composition was diluted into solutions having a series of concentrations as shown in Table 2-1. All the experimental operations were performed in the BSL-3 laboratory.

As shown in Table 2-1, the phillyrin/phillygenin composition, even having a low concentration, can effectively inhibit HCoV-229E. By calculation, the half maximal inhibitory concentration (IC₅₀) is 64.53 µg/mL (while the inhibitory effect of pure phillyrin and pure phillygenin showed by the study at the same time is worse). The combination of phillyrin and phillygenin in the composition has a synergistic effect, so it is expected to be used to treat diseases caused by HCoV-229E.

**Table 2-1**

| Drug concentration (µg/mL) | Inhibition (%) |
|---|---|
| 500.000 | 88.75±2.50 |
| 250.000 | 83.75±4.79 |
| 125.000 | 75.00±4.08 |
| 62.500 | 47.50±6.45 |
| 31.250 | 28.75±4.79 |
| 15.625 | 11.25±4.79 |

### Third Embodiment: Study on Molecular Mechanism of Phillyrin and its Derivatives to COVID-19 Virus

### I. Experimental Background

Spike protein (S protein) on the surface of COVID-19 virus and hydrolase of COVID-19 virus (3-chymotrypsin-like cysteine protease, abbreviated as 3CL pro protein) are very important parts in the life cycle of COVID-19 virus. The life cycle of COVID-19 virus involves that the S protein on the surface of COVID-19 is combined with angiotensin-converting enzyme 2 (ACE2), the cell is invaded through cell endocytosis so that the genetic material RNA is released, and the genetic material RNA is successfully replicated with the participation of the 3CL pro protein to produce a virus.

Virtual screening is mainly based on molecular docking technology, which simulates the process of drug screening by using a computer. First, for performing such screening, it is necessary to know the molecular structure of the target on which the drug acts, the binding ability of small molecules in the compound library to the target is calculated by means of molecular simulation, and the physiological activity of candidate compounds is predicted.

In this study, Glide docking technique was used to perform virtual screening of the optimal binding effect of phillyrin (KD-1) and its derivatives on 3CL pro target protein. Based on the screening results, the binding of KD-1 and its derivatives to the target protein in vitro was studied, and the molecular interaction and binding mode as well as the structure-activity relationship of KD-1 and its derivatives were elucidated. The mechanism in which KD-1 and its derivatives act as a potential COVID-19 inhibitor molecular was revealed by the assay of activity of KD-1 and its derivatives on the target protein.

### II. Experimental methods

### 1 Molecular docking of KD-1 and its derivatives to target proteins

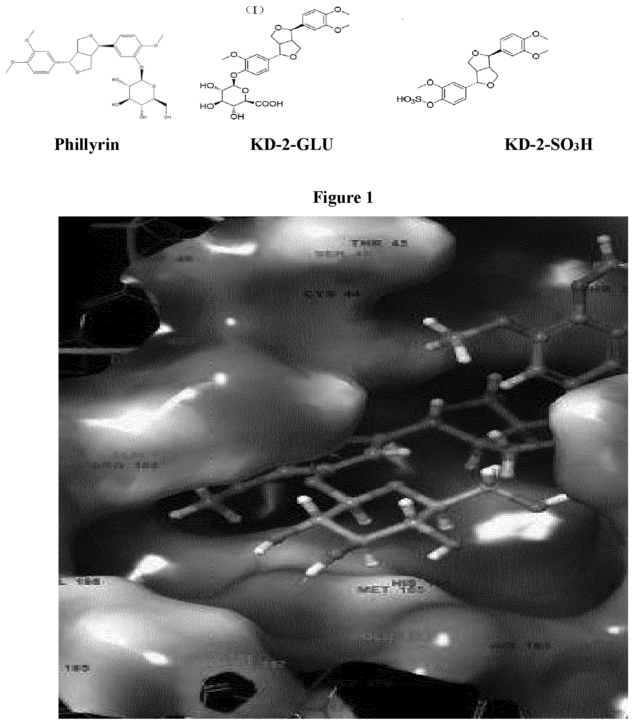

**(1) System preparation:** The 2.16 Å crystal structure of SARS-CoV-2 main protease (3CL pro protein) was obtained from the Protein Data Bank (PDB ID: 6LU7). The structure of the enzyme was pretreated with the Protein Preparation Wizard in Schrodinger, crystalline water was eliminated and missing hydrogen/side-chain atoms were added, and appropriate charges and protonation states were assigned to acidic and basic amino acid residues at pH 7.0, energy minimization of the structure of the enzyme was performed using the OPLS-2005 force field, and finally the active site of the protein was predicted using Sitemap. Meanwhile, the structural conformation of phillyrin and its derivatives were optimized by LigPrep module in Schrodinger.
**(2) Molecular docking:** Molecular docking-based virtual screening was performed using the Glide workflow of Maestro 11.5. Docking calculations on phillyrin and its derivatives (Figure 1) were performed by using the "ultra-precision" mode (XP) of Glide.
**(3) Docking score:** The "Glide-score" score function value was used to evaluate the interaction between a small molecule and the target protein. This function takes interactions such as hydrogen bond, hydrophobic interaction, van der Waals force, or the like into account. The larger the absolute value of the function, the more stable the docking complex including the small molecule and the target protein and the better the matching binding effect.
**(4) Analysis of targets:** Based on the docking results of KD-1 and its derivatives with the key target protein of the novel coronavirus, key amino acids involved in the interaction between KD-1 and its derivatives and the key target protein of the novel coronavirus were analyzed to provide a theoretical basis and important reference for clinical prevention, diagnosis and treatment of the novel coronavirus infection.

### 2 Expression and purification of protein

**(1) Cloning:** Full-length gene encoding SARS-CoV-2 3CL pro protein (UniprotKB - P0DTD1, residues 3264-3569) had been optimized and synthesized with respect to the E. coli expression system (Wuhan Genecreate Bioengineering Co., Ltd.).
**(2) Expression:** Expression plasmids were transformed into E. coli BL21 (DE3) cells and then cultured with LB medium containing 100 µg/mL ampicillin at 37°C. When the cells were grown to have an OD600 nm value of 0.6-0.8, 0.5 mM IPTG was added to the cell culture to induce the expression at 180 rpm and 30°C. After 10 hours, the cells were harvested by centrifugation at 3,000 g.
**(3) Purification:** Cell precipitation was resuspended in lysis buffer (20 mM Tris-HCl pH 8.0, 150 mM NaCl, 2mM BME), lysed by ultrasonication, and centrifuged at 13,000 g for 30 min. The supernatant was loaded onto a Ni-NTA affinity column, followed by washing with resuspension buffer of 20 mM imidazole, and His-tagged 3CL pro protein was eluted with lysis buffer of 300 mM imidazole (50 mM Tris-HCl pH 7.0, 150 mM NaCl). The 3CL pro protein was further purified by ion exchange chromatography. The 3CL pro protein was finally obtained as a mixture of monomer and dimer and preserved in solution.

### 3 Spectroscopy experiment

In this study, change in the structure of protein was investigated by using JASCO-V560 UV-Vis spectrophotometer. The UV absorption spectrum of the protein has two absorption peaks. A strong absorption peak at 210 nm reflects the frame conformation of the protein, which attributes to n-π ^{∗} transition of C=O in the peptide bond. A weak absorption peak at 280 nm attributes to π-π ^{∗} transition due to absorption of light by aromatic amino acids (Trp, Tyr and Phe).

In this experiment, the UV-Vis absorption spectra of protein solution and phillyrin-protein mixture solution were scanned in a wavelength range of 200-700 nm with a buffer solution as a reference. 2 mL of Gel Buffer (50 mM Tris-HCl pH 7.0, 150 mM NaCl) was added to a quartz cuvette, 10 µL of 3CL pro protein was added at a concentration of 10⁻⁵ M, and the absorbance value thereof was measured, and meanwhile, 10 µL of phillyrin and its derivatives were added at a concentration of 10⁻⁴ M, and the changes in the absorbance value were measured.

### III. Experimental results

The life cycle of COVID-19 virus involves that the S protein on the surface of COVID-19 is combined with angiotensin-converting enzyme 2 (ACE2), the cell is invaded through cell endocytosis so that the genetic material RNA is released, and the genetic material RNA is successfully replicated with the participation of the 3CL pro protein to produce a virus. In this experiment, we explored a compound that can block the participation of the 3CL pro protein in replication of the genetic material RNA, and then developed anti-COVID-19 drugs. In this experiment, we selected the optimal target site based on the crystal structure of 3CL pro protein and performed virtual screening on KD-1 and its derivative (KD-2-GLU and KD-2-SO₃H) compounds. The binding scores of KD-1, KD-2-GLU and KD-2-SO₃H compounds to 3CL pro protein site were obtained, thereby proving targeting of the compounds.

By calculation and analysis, five potential active sites were found in the three-dimensional structure of 3CLpro protein, among which site 1 is a site where the drug molecule is easy to bind. KD-1 was docked to five potential active sites of 3CLpro protein, and all five active sites were successfully docked, with docking scores shown in Table 3-1. The Glide Score of KD-1 at active site 1 of 3CLpro protein was significantly higher than that of counterparts at other active sites of 3CLpro protein, and active site 1 of 3CLpro protein was selected as a theoretical value of molecular docking. The analysis and calculation of KD-2-GLU and KD-2-SO₃H compounds were the same as above.

**Table 3-1 Docking scores of KD-1 with five potential active sites of 3CLpro protein**

| **Small molecule** | **3CLpro protein number** | **Active site** | **Docking score** |
|---|---|---|---|
| KD-1 | 6lu7 | Site1 | -9.461 |
| KD-1 | 6lu7 | Site2 | -3.954 |
| KD-1 | 6lu7 | Site3 | -4.611 |
| KD-1 | 6lu7 | Site4 | -3.342 |
| KD-1 | 6lu7 | Site5 | -4.141 |

The active site at which KD-1, KD-2-GLU, and KD-2-SO₃H have the optimal activity on 3CLpro protein was selected. The binding ability of small molecules to 3CLpro protein site was evaluated by "Glide Score". The higher the absolute value, the more stable the binding therebetween, the better the matching. Docking scores of KD-1, KD-2-GLU, and KD-2-SO₃H compounds to the active site of the COVID-19 target protein are shown in Table 3-2. Table 3-2 shows that the theoretical docking scores of KD-1, KD-2-GLU, and KD-2-SO₃H compounds to the core protein 3CL pro of COVID-19, which is involved in RNA replication, are significant. KD-1 and KD-2-GLU had high docking scores, indicating that these compounds have different degrees of targeting to the core protein 3CL pro.

**Table 3-2 Summary of docking scores of KD-1 and its derivatives to 3CLpro target protein site of COVID-19**

| **Number** | **Compound** | **3CLpro Protein Number** | **Docking score** |
|---|---|---|---|
| 1 | KD-1 | 6LU7 | -9.461 |
| 2 | KD-2-GLU | 6LU7 | -8.222 |
| 3 | KD-2-SO₃H | 6LU7 | -5.886 |

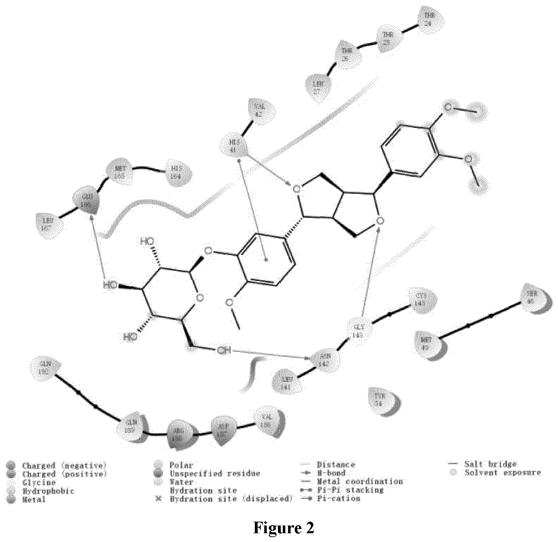

From the three-dimensional simulation diagram and plane view of molecular docking of KD-1 to 3CLpro target protein, it can be seen that the KD-1 compound entered the molecular pocket of the protein (upper part of Figure 2). KD-1 was bound to the 3CL pro protein by forming π-π conjugation with amino acid His41 residue in the active site of the 3CL pro protein, forming 3 hydrogen bonds with peptide bonds of Gly143, Asn142, and Glu166, and interacting with Met165 via hydrophobic interaction (lower part of Figure 2). The results indicated that various amino acid residues, such as His, Gly, Asn, Glu, Met or the like, could sterically match the structure of KD-1 to determine the conformation of the complex. It was speculated that the KD-1 compound could target the 3CL pro protein and prevent the 3CL pro protein from participating in RNA replication of COVID-19 for producing virus.

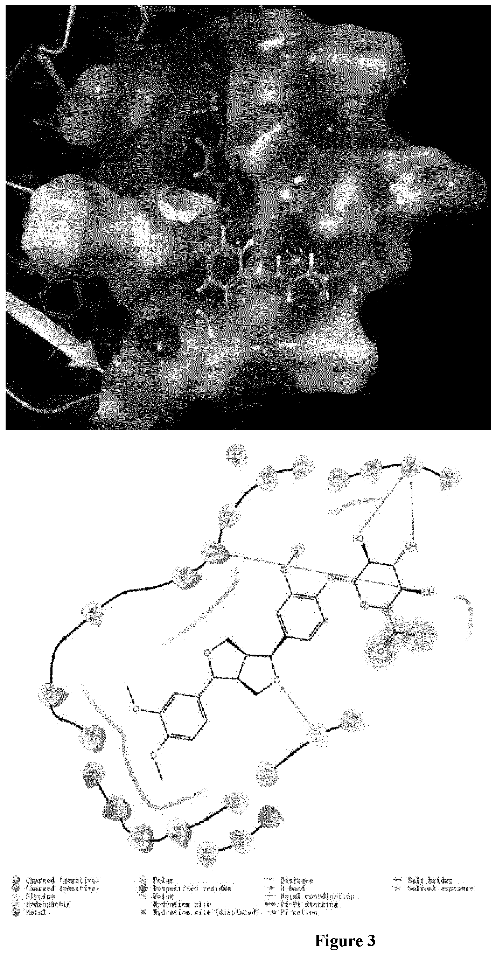

From the three-dimensional simulation diagram and plane view of molecular docking of KD-2-GLU to 3CLpro target protein, it can be seen that the KD-2-GLU compound entered the molecular pocket of the protein (upper part of Figure 3). KD-2-GLU was bound to the 3CL pro protein by forming 3 hydrogen bonds with peptide bonds of Gly143, Thr43, and Thr25 in the active site of the 3CL pro protein, and interacting with Tyr54 via hydrophobic interaction (lower part of Figure. 3). The results indicated that various amino acid residues, such as Gly, Thr, Tyr or the like, could sterically match the structure of KD-2-GLU and thus determine the conformation of the complex. It was speculated that the KD-2-GLU compound could target the 3CL pro protein and prevent the 3CL pro protein from participating in RNA replication of COVID-19 for producing virus.

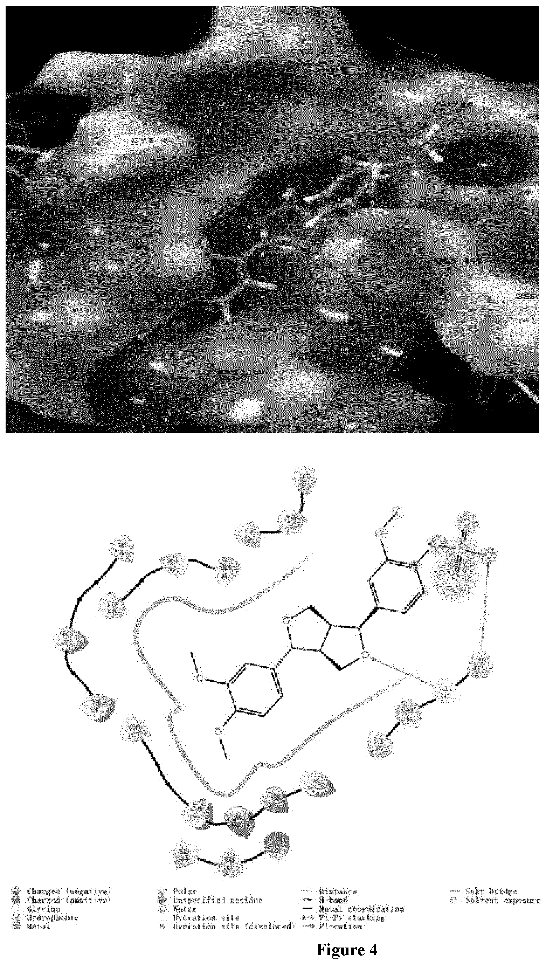

From the three-dimensional simulation diagram and plane view of molecular docking of KD-2-SO₃H to 3CLpro target protein, it can be seen that the KD-2-GLU compound entered the molecular pocket of the protein (upper part of Figure 4). KD-2-SO₃H interacted with Gly143 and Asn142 by forming hydrogen bond, and interacted with Tyr54, Val42, Cys44, and Cys145 via hydrophobic interaction (lower part of Figure 4). The results indicated that various amino acid residues, such as Gly, Asn, Val or the like, could sterically match the structure of KD-2-SO₃H and thus determine the conformation of the complex.

The interaction between KD-1 and its derivatives and the active site of 3CLpro protein is shown in Table 3-3. The results show that, in addition to the hydrophobic interaction, the interactions between phillyrin and two derivatives and the active site of 3CLpro protein further include hydrogen bonds and π-π conjugation. The interactions via hydrogen bonds and π-π conjugation may contribute to stabilization of the complex. The KD-1, KD-2-GLU and KD-2-SO₃H compounds were found to interact with 3CLpro protein via hydrogen bond at a single GLY143, with hydrogen atom spacings of 2.22 Å, 2.22 Å and 2.06 Å, respectively. However, hydrophobic and electrostatic interactions play an important role in the process of binding KD-1, KD-2-GLU and KD-2-SO₃H compounds to 3CLpro protein. Different compounds bind to different residues of 3CLpro protein, which explains the difference of their binding results.

**Table 3-3 Interaction between KD-1 and its derivatives and 3CLpro protein**

| Small molecule | Interaction | **Key amino acid** |
|---|---|---|
| KD-1 | Hydrogen bonding | **HIS41** |
| | Hydrophobic interaction | **HIS41, GLY143, ASN142, GLU166** |
| | π-π conjugation | **HIS41** |
| KD-2-GLU | Hydrogen bonding | **THR43, THR25, GLY143** |
| | Hydrophobic interaction | **TYR54** |
| KD-2-SO₃H | Hydrogen bonding | **GLY143, ASN142** |
| | Hydrophobic effect | **VAL42, CYS44, TYR54, CYS145** |

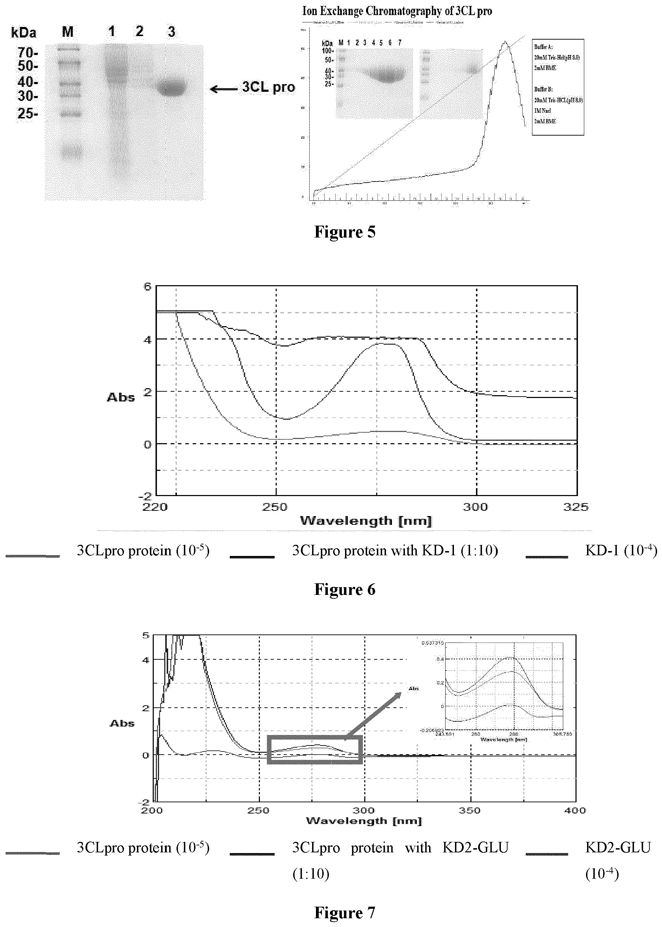

The fragmented protein was purified using a nickel column and was found to have a molecular weight of 34 kDa (left part of Figure 5). Ion exchange chromatography was performed and then SDS PAGE was performed to detect sample 3CL pro protein, with a protein concentration of 65 mg/mL (right part of Figure 5).

Based on the results of virtual screening using molecular docking, the binding of KD-1, KD-2-GLU and KD-2-SO₃H compounds to 3CLpro protein was tested in vitro by UV-Vis absorbance detection method, further proving that the compounds can target 3CLpro protein. Figure 6 shows the absorbance of KD-1, 3CLpro protein and a mixture thereof detected by UV-Vis spectroscopy. The results showed that 3CL pro protein had an absorption peak at 278 nm, and the absorbance was increased and the peak shifted after KD-1 was added. KD-1 interacts with 3CL pro protein and may inhibit the activity of the enzyme. KD-1 may form a new complex with 3CL pro protein, and KD-1 may be bonded to 3CL pro protein through hydrogen bond or hydrophobic interaction.

Figure 7 shows the absorbance of KD2-GLU, 3CLpro protein and a mixture thereof detected by UV-Vis spectroscopy. The results showed that 3CL pro protein had an absorption peak at 278 nm, and the absorbance was increased and the peak shifted after KD2-GLU was added. KD2-GLU interacts with 3CL pro protein and may inhibit the activity of the enzyme. KD2-GLU may form a new complex with 3CL pro protein, and they may be bonded through hydrogen bond and hydrophobic interaction.

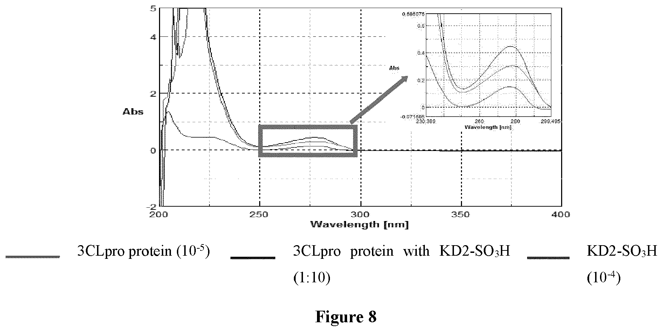

Figure 8 shows the absorbance of KD2-SO₃H, 3CLpro protein and a mixture thereof detected by UV-Vis spectroscopy. The results showed that the absorbance was increased and the peak of 3CL pro protein shifted after KD2-SO₃H was added. KD2-SO₃H interacts with 3CL pro protein and may inhibit the activity of the enzyme. KD2-SO₃H may be bonded to 3CL pro protein through hydrogen bond or hydrophobic interaction, and they may form a new complex.

### IV. Experimental Conclusion

The molecular docking results showed that the theoretical docking scores of KD-1, KD-2-GLU and KD-2-SO₃H compounds to 3CL pro protein, as a core protein of COVID-19 involved in RNA replication, were significant, and in which, KD-1 and KD-2-GLU had high docking scores. The analysis showed that KD-1, KD-2-GLU and KD-2-SO₃H compounds were bonded to peptide bonds in the active site of 3CL pro protein through hydrogen bond and hydrophobic interaction, and formed π-π conjugation with amino acid residues. The theoretical data could prove that these compounds could change the structure of 3CL pro protein. Results of spectroscopic experiments in vitro showed that KD-1, KD-2-GLU and KD-2-SO₃H compounds all shifted and increased the characteristic peak of 3CL pro protein at 278 nm, and it was most significant for KD-1 compound. Therefore, each of KD-1, KD-2-GLU and KD-2-SO₃H compounds can target 3CL pro protein, may prevent 3CL pro protein of COVID-19 virus from participating in viral replication, and thus may be a potential inhibitor targeting COVID-19 virus.

### Fourth Embodiment: In Vivo Study on Efficacy against Novel Coronavirus

### I. Experimental Materials

Mice: hACE2 mice, 6-7 weeks old, 20-40 g, 120 males in total, laboratory animal supplier: Jiangsu Gempharmatech Co., Ltd.; laboratory animal production license: SCXK (Su) 2018-0008; laboratory animal certificate No.: No.320727201100243581; diets supplier: Jiangsu Medicience Biopharmaceutical Co., Ltd.

Drug: phillyrin/phillygenin composition, wherein the weight ratio of phillyrin to phillygenin is 90: 10 (Dalian Fusheng Natural Medicine Development Co., Ltd.).

### II. Experimental methods

### (1) Protective effect of drug on disease progression in COVID-19 mice

The hACE2 transgenic C57BL/6 mice were divided into normal group, SARS-CoV-2 infected group, 80 mg/kg dosage administrating group, 40 mg/kg dosage administrating group, and positive control group (Remdesivir 50 mg/kg), 8 mice in each group. Except for administrating PBS to the mice in the normal group by nasal drip, the mice in other groups were infected with 10⁵ PFU of SARS-CoV-2 virus by nasal drip. Two hours after infection, phillyrin was intragastrically administrated to the mice in the administrating groups, once a day, for 5 consecutive days. Changes in body weight were recorded every day after infection, and 5-day mortality was calculated.

### (2) Study on the effect of drugs on induced excessive inflammation of COVID-19 infected mice

ACE2 transgenic C57BL/6 mice were divided into normal group, SARS-CoV-2 infected group, 80 mg/kg dosage administrating group, 40 mg/kg dosage administrating group, and positive control group (Remdesivir 50 mg/kg). Except for administrating PBS to the mice in the normal group by nasal drip, the mice in other groups were infected with 10⁵ PFU of SARS-CoV-2 virus by nasal drip. Two hours after infection, phillyrin was intragastrically administrated to the mice in the administrating groups, once a day, for 5 consecutive days. On the fifth day after infection, the animals were dissected to take lungs for tissue homogenization to test the viral titer. Total RNA was extracted from the supernatant of lung tissue homogenate by Trizol method, and the mRNA expression of related inflammatory factors was detected by RT-qPCR.

### III. Experimental results

### (1) Study results of death protection of drug against novel coronavirus

The results of death protection are shown in Table 4-1. The results showed that the protective effects of administration in two dosages (80 mg/kg and 40 mg/kg) on the mice infected with novel coronavirus were 87.5% and 42.86%, respectively; 71.43% of the mice in the virus infected group died, and the 80 mg/kg dosage administrating group had a death protection rate comparable to that of the Remdesivir group.

**Table 4-1 Test results of death protection of mice infected with novel coronavirus (n=3)**

| **Group** | **Mean survival days (days)** | **Survival rate (%)** | **Mortality rate (%)** |
|---|---|---|---|
| Normal group | 5.00±0.00 | 100 | 0.00 |
| SARS-CoV-2 infected group | 4.29±0.49 | 28.57 | 71.43 |
| Administrating 80 mg/kg | 4.88±0.35 | 87.50 | 12.50 |
| Administrating 40 mg/kg | 4.43±0.53 | 42.86 | 57.14 |
| Remdesivir 50 mg/kg | 4.86±0.38 | 87.50 | 12.50 |

### (2) Test results of viral titer in lung tissues of novel coronavirus-infected mice administrated with drugs

As shown in Table 4-2, by administration in two dosages (80 mg/kg and 40 mg/kg), viral titers in lungs of infected mice were significantly reduced, and the difference in inhibition of viral titer in lung was not statistically significant between the 80 mg/kg dosage administrating group and the Remdesivir group.

**Table 4-2 Test results of viral titer in lung tissues of novel coronavirus-infected mice (x̅±s, n=3)**

| **Group** | **Viral titer in lung (Log10)** |
|---|---|
| Normal group | - |
| SARS-CoV-2 infected group | 3.14±0.41 |
| Administrating 80 mg/kg | 1.99±0.38^{∗∗∗} |
| Administrating 40 mg/kg | 2.54±0.32^{∗∗∗} |
| Remdesivir 50 mg/kg | 1.68±0.45^{∗} |

| | |
|---|---|
| **Note: Compared with virus infected group, ^{∗} P < 0.05, ^{∗∗}P < 0.01, ^{∗∗∗}P < 0.001** | |

### (3) Test results of induced excessive inflammation of novel coronavirus-infected mice administrated with drugs

Table 4-3 shows test results of inflammatory indicators of the infected mice. On the fifth day after infection with novel coronavirus, expressions of IL-1β, IFN-γ, MCP-1 and IFN-α of the infected group were significantly increased. With drug intervention, administration in two dosages (80 mg/kg and 40 mg/kg) had inhibitory effects on IL-1β, IFN-γ, MCP-1 and IFN-α inflammatory factors. The 80 mg/kg dosage administrating group had an ability to inhibit the overexpression of inflammatory mediators, which is superior to Remdesivir.

**Table 4-3 Effect of drug on mRNA expression of IL-1β, IFN-γ, MCP-1 and IFN-α in lung homogenate of infected mice (x̅± s, n=3)**

| Group | IL-1β | IFN-γ | IFN-α | MCP-1 |
|---|---|---|---|---|
| Normal group | 1.00±0.001^{∗∗∗} | 1.16±0.29^{∗∗∗} | 1.03±0.045^{∗∗∗} | 1.00±0.00^{∗∗∗} |
| SARS-CoV-2 infected group | 43.42±10.97 | 432.60±116.11 | 246.63±49.28 | 14.72±4.99 |
| Remdesivir 50 mg/kg | 7.41±5.86^{∗∗∗} | 173.79±84.84^{∗} | 69.99±53.20^{∗∗} | 7.31±5.14^{∗∗} |
| Administrating 80 mg/kg | 6.14±3.71^{∗∗∗} | 51.56±32.33^{∗∗∗} | 50.22±16.94^{∗∗} | 1.80±0.73^{∗∗∗} |
| Administrating 40 mg/kg | 11.00±3.84^{∗∗∗} | 228.07±180.55 | 157.04±42.06^{∗∗} | 6.89±2.39^{∗∗} |

| | | | | |
|---|---|---|---|---|
| **Note: Compared with virus infected group, * P** < **0.05, **P** < **0.01, ***P** < **0.001** | | | | |

### IV. Conclusion

The above experimental results show that the drug of the present invention has a significant protective effect on the death of mice infected with novel coronavirus, with a protective rate of up to 87.5% against the death of mice infected with novel coronavirus, which is comparable to that of Remdesivir. The drug of the present invention can significantly inhibit the virus titer in lung tissues of mice infected with novel coronavirus. It can also inhibit the mRNA expression of excessive inflammatory factors IL-1β, IFN-α, MCP-1 and IFN-γ induced by novel coronavirus infection. Therefore, the drug of the present invention plays a role in the treatment of novel coronavirus infection.

## Claims

1. A use of phillyrin and/or a derivative thereof in preparation of an agent for inhibiting 3CLpro protein of COVID-19 virus.

2. The use of claim 1, wherein the derivative is KD-2-GLU or KD-2-SO₃H.

3. A method for inhibiting 3CLpro protein of COVID-19 virus, comprising a step of contacting 3CLpro protein of COVID-19 virus with phillyrin and/or a derivative thereof.

4. The method of claim 3, wherein the derivative is KD-2-GLU or KD-2-SO₃H.

5. A use of a phillyrin/phillygenin composition in preparation of a drug against COVID-19 virus or a drug for treating a disease caused by COVID-19 virus.

6. The use of claim 5, wherein the weight ratio of phillyrin to phillygenin in the phillyrin/phillygenin composition is 2~98: 2~98.

7. The use of claim 5, which is a use of the phillyrin/phillygenin composition as a sole active pharmaceutical ingredient.

8. The use of claim 5, wherein the drug is present in a form of tablet, capsule, pill, powder, granule, syrup, solution, emulsion, injection, spray, aerosol, gel, cream, cataplasmata, adhesive plaster or emplastrum.

9. The use of claim 5, wherein the phillyrin/phillygenin composition inhibits 3CLpro protein of COVID-19 virus.

10. A method against COVID-19 virus or a method for treating a disease caused by COVID-19 virus, comprising a step of administrating an effective amount of a phillyrin/phillygenin composition to a patient in need.

11. A method against COVID-19 virus or a method for treating a disease caused by COVID-19 virus, comprising a step of administrating an effective amount of a drug including a phillyrin/phillygenin composition to a patient in need.

12. The method of claim 10 or claim 11, wherein the weight ratio of phillyrin to phillygenin in the phillyrin/phillygenin composition is 2~98: 2~98.

13. The method of claim 10 or claim 11, wherein the phillyrin/phillygenin composition is used as a sole active pharmaceutical ingredient.

14. The method of claim 10 or claim 11, wherein the drug is present in a form of tablet, capsule, pill, powder, granule, syrup, solution, emulsion, injection, spray, aerosol, gel, cream, cataplasmata, adhesive plaster or emplastrum.

15. The method of claim 10 or claim 11, wherein the phillyrin/phillygenin composition inhibits 3CLpro protein of COVID-19 virus.

16. A phillyrin/phillygenin composition against COVID-19 virus, or a phillyrin/phillygenin composition for treating a disease caused by COVID-19 virus, or a phillyrin/phillygenin composition for inhibiting 3CLpro protein of COVID-19 virus.

17. The composition of claim 16, wherein the weight ratio of phillyrin to phillygenin in the phillyrin/phillygenin composition is 2~98: 2~98.

18. A drug against COVID-19 virus, or a drug for treating a disease caused by COVID-19 virus, or a drug for inhibiting 3CLpro protein of COVID-19 virus, wherein the drug comprises a phillyrin/phillygenin composition.

19. The drug of claim 18, wherein the weight ratio of phillyrin to phillygenin in the phillyrin/phillygenin composition is 2~98: 2~98.

20. The drug of claim 18, wherein the phillyrin/phillygenin composition is used as a sole active pharmaceutical ingredient.

21. The drug of claim 18, wherein the drug is present in a form of tablet, capsule, pill, powder, granule, syrup, solution, emulsion, injection, spray, aerosol, gel, cream, cataplasmata, adhesive plaster or emplastrum.
